# EUROPÄISCHE PATENTSCHRIFT

(11) **EP 0 960 884 B1**
(45) Veröffentlichungstag und Bekanntmachung des Hinweises auf die Patenterteilung: **09.10.2002**
(21) Anmeldenummer: 99117097.8
(22) Anmeldetag: 03.11.1993
(51) Int. Cl.: C07D 473/10

(54) **Verfahren zur Gewinnung von 3,7-Dialkylxanthinen aus 3-Alkylxanthinen**
Process for the production of 3,7-dialkylxanthins from 3-alkylxanthins
Procédé pour l'obtention de 3,7-dialkylxanthines à partir de 3-alkylxanthines

(30) Priorität: 10.11.1992 DE 4237814
(43) Veröffentlichungstag der Anmeldung: 01.12.1999
(62) Teilanmeldung aus: 93117817.2
(73) Patentinhaber: Aventis Pharma Deutschland GmbH, 65929 Frankfurt am Main (DE)
(72) Erfinder: Korb, Gerhard, 63512 Hainburg (DE); Flemming, Hans-Wolfram, Dr., 61250 Usingen (DE)

(56) Entgegenhaltungen:
- EP-A- 0 019 165
- EP-A- 0 319 854
- DD-A- 222 026
- DE-C- 864 869
- DATABASE WPI Week 9303 Derwent Publications Ltd., London, GB; AN 93-025154 XP002116808 & RO 101 894 A (INST. CHIM. CLUJ-NAPOCA)
- CHEMICAL ABSTRACTS, vol. 113, no. 5, 30. Juli 1990 (1990-07-30) Columbus, Ohio, US; abstract no. 40721u, XP002116803 & CS 263 595 A (RYBAR A. ET AL.)
- CHEMICAL ABSTRACTS, vol. 115, no. 25, 23. Dezember 1991 (1991-12-23) Columbus, Ohio, US; abstract no. 279704u, XP002116804 & CS 267 100 A (RYBAR A. ET AL.)
- CHEMICAL ABSTRACTS, vol. 113, no. 5, 30. Juli 1990 (1990-07-30) Columbus, Ohio, US; abstract no. 40720t, XP002116805 & CS 263 200 A (RYBAR A. ET AL.)
- CHEMICAL ABSTRACTS, vol. 114, no. 19, 13. Mai 1991 (1991-05-13) Columbus, Ohio, US; abstract no. 185144p, XP002116806 & CS 267 796 A (RYBAR A. ET AL.)
- CHEMICAL ABSTRACTS, vol. 117, no. 9, 31. August 1992 (1992-08-31) Columbus, Ohio, US; abstract no. 90053f, XP002116807 & CS 270 545 A (RYBAR A. ET AL.)

## Beschreibung

Verfahren zur Gewinnung von 3,7-Dialkylxanthinen aus 3-Alkylxanthinen 3,7-Dialkylxanthine sind Verbindungen der Formel I.

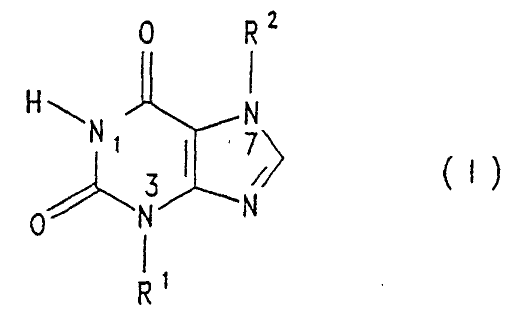

Die unsubsituierten oder auch an Position 1 substituierten 3,7-Dialkylxanthine sind hauptsächliche Vor- oder Zwischenprodukte zur Herstellung von Arzneimitteln. So kann beispielsweise die Verbindung 3,7-Dimethylxanthin (Theobromin) als Vorprodukt zur Herstellung des Arzneimittelwirkstoffs Pentoxifyllin (US 3 737 433) der Formel VIII 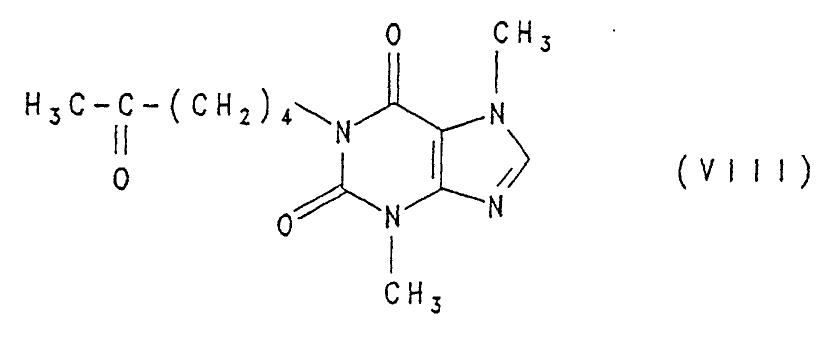 und ähnlicher Verbindungen, die vasotherapeutische Eigenschaften besitzen, verwendet werden.

Zur Gewinnung von spezifikationsgerechtem Pentoxifyllin (VIII) muß Theobromin in genügend hoher Reinheit eingesetzt werden. Störend wirken insbesondere die Ausgangsverbindung 3-Methylxanthin (II, R¹ = CH₃), die übermethylierte Verbindung Coffein (IX) und die isomere Verbindung Theophyllin (X) 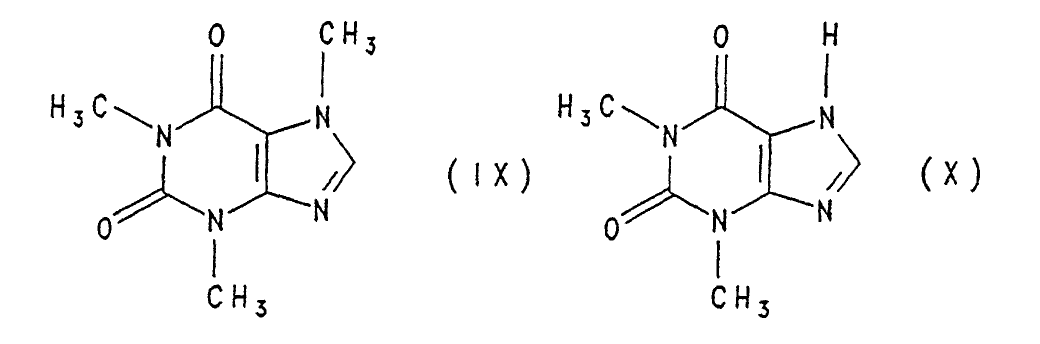 sowie färbende Verunreinigungen.

In der Literatur sind Verfahren zur Herstellung der Verbindung der Formel (I) bekannt. Dabei kommen im wesentlichen als Alkylierungsmittel Dimethylsulfat, Dimethylcarbonat und Methylchlorid, Ethylchlorid, Propylchlorid, Diethylsulfat, Dipropylsulfat, Diethylcarbonat oder Dipropylcarbonat zum Einsatz.

Die Verfahren mit Dimethylsulfat als Methylierungsmittel zur Herstellung von Theobromin aus 3-Methylxanthin sind in der Patentschrift DD 222 026 zusammengefaßt. Je nach angewandter Methylierungsmethode erhält man Theobromin in Ausbeuten von 65 bis 76 % der Theorie, bezogen auf reines Theobromin. Jedoch ergeben sich bei den Verfahren mit hohen Theobromin-Ausbeuten meist noch größere Mengen an Nebenverbindungen, die wegen der Qualitätsanforderungen für die Herstellung von Arzneimittelwirkstoffen durch einen zusätzlichen Reinigungsgang entfernt werden müssen.

Das Verfahren mit Dimethylcarbonat als Methylierungsmittel (DE 3 741 883) ist ein Hochdruckverfahren, das nur in speziellen Autoklaven durchgeführt werden kann, die Drücke von 80 bis 160 bar aushalten. Die erzielten Ausbeuten liegen dabei zwischen 65,8 und 73,2 % der Theorie an reinem Theobromin mit einem Gehalt von mindestens 99,6 %, der durch HPLC ermittelt wurde.

Das Verfahren mit Methylchlorid als Methylierungsmittel zur Herstellung von Theobromin (CS 267 100) in einem wäßrigen oder wäßrig/alkoholischen Milieu weist hohe Ausbeuten auf. Jedoch handelt es sich hier um ein Theobromin, das sehr stark mit Nebenprodukten verunreinigt sein muß, da die angegebenen Schmelzpunkte sehr weit vom Schmelzpunkt des reinen Theobromins entfernt liegen. Ein Gemisch aus reinem Theobromin und etwa 5 % 3-Methylxanthin weist beispielsweise immer noch einen um ca. 100 °C höher liegenden Schmelzpunkt auf als nach CS 267 100 hergestelltes Theobromin. Man muß deshalb davon ausgehen, daß noch erhebliche Mengen Coffein (IX) und ggf. Theophyllin (X) im isolierten Produkt enthalten sind, die durch Reinigung entfernt werden müssen. Der Ausbeuteverlust bei der weiteren Aufarbeitung zu reinem Theobromin dürfte ganz erheblich sein.

Es ist ferner ein Verfahren zur Herstellung von 3-Methyl-7-propylxanthin (CS 267 796) bekannt, wobei das Verfahren durch Zusatz kleiner Mengen an Katalysatoren für Zwischenphasenübertragung wie Tetraalkylammoniumsalze oder Dimethylbenzylalkylammoniumsalze mit 8 bis 18 Kohlenstoffatomen verbessert werden soll. Jedoch zeigen die Beispiele, daß der Zusatz von etwa 0,26 mMol Dimethylbenzylalkylammoniumbromid zu einer Verminderung der Ausbeute um 5 % führt, wobei die Schmelzpunkte der mit oder ohne Katalysator hergestellten Produkte gleich sind.

Ferner zeigen die in der Patentschrift CS 267 100 aufgeführten Beispiele, daß bei größeren Ansätzen die Ausbeute kleiner ist. Diese Tatsache wurde auch in der Patentschrift DD 222 026 beschrieben. Eigene Versuche bei denen die Ansatzgröße verhundertfacht wurde, zeigen unter den in CS 267 796 beschriebenen Bedingungen eine deutliche Verringerung der Ausbeute.

Aufgabe der vorliegenden Erfindung ist es, ein Verfahren zu finden, durch das selektiv 3,7-Dialkylxanthine aus 3-Alkylxanthinen in hohen Ausbeuten bei gleichzeitig sehr hohen Reinheiten und sehr guten Raum/Zeitausbeuten herstellbar sind.

Die Erfindung betrifft daher ein Verfahren zur Gewinnung von 3,7-Dialkylxanthinen der Formel I 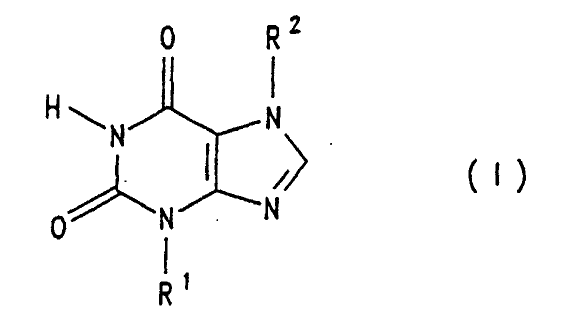 wobei R¹ und R² unabhängig voneinander für (C₁-C₆)-Alkyl, geradkettig oder verzweigt, stehen,
aus 3-Alkylxanthin der Formel II 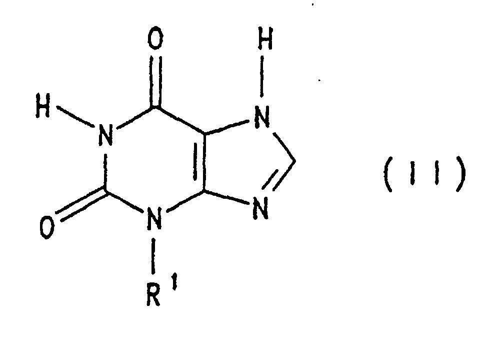 in der R¹ für (C₁-C₆)-Alkyl, geradkettig oder verzweigt, steht,
daß zuerst in wäßriger Phase mit einem basischen Mittel in sein Salz überführt und anschließend alkyliert wird, dadurch gekennzeichnet, daß man das erhaltene Salz in Gegenwart von mindestens einer in Wasser schwer oder nicht löslichen quartären Ammoniumverbindung der Formel III 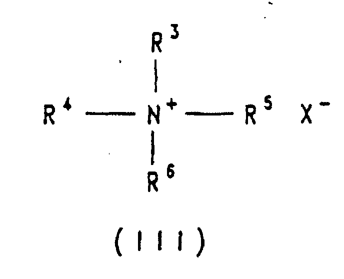 wobei
- R³ bis R⁶: gleich oder verschieden sind und unabhängig voneinander für
a) (C₁-C₂₀)-Alkyl, geradkettig oder verzweigt,
b) Benzyl oder
c) Phenyl stehen und
- X: für ein Anion aus der Gruppe Chlorid, Bromid, Hydrogensulfat oder Hydroxid steht, in einem Zweiphasengemisch enthaltend zwei flüssige Phasen, worin die eine Phase wasser und die andere Phase die Verbindung der Formel III mit einem Alkylierungsmittel mit 1 bis 6 Kohlenstoffatomen im Alkylteil umsetzt,
und auf 100 Mol 3-Alkylxanthin der Formel II 5 bis 100 Mol der Verbindung der Formel III eingesetzt werden.
Gegebenenfalls wird die Alkylierung in Gegenwart eines linearen Polyethers der Formel V

R¹¹ - O - (Y)ₙ - R¹² (V)

wobei
R¹¹ und R¹² gleich oder verschieden sind und unabhängig voneinander für (C₁-C₈)-Alkyl stehen,
   - Y: für eine Rest aus der Gruppe
   a) -CH₂-CH₂-O- oder
   b) -CH₂-CH₂-CH₂-O-
und n für eine ganze Zahl von 1 bis 8 steht,
durchgeführt.

Bevorzugt werden in dem erfindungsgemäßen Verfahren 3,7-Dialkylxanthine der Formel I, wobei R¹ und R² unabhängig voneinander für (C₁-C₃)-Alkyl stehen, hergestellt.

Für das Verfahren besonders bevorzugte 3,7-Dialkylxanthine sind
- 3,7-Dimethylxanthin: (= Verbindung der Formel I mit R¹ = R² = -CH₃
- 3-Ethyl-7-propylxanthin: (= Verbindung der Formel I mit R¹ = -CH₂-CH₃ und R² = -CH₂-CH₂-CH₃)
- 3-Methyl-7-propylxanthin: (= Verbindung der Formel I mit R¹ = CH₃ und R² = -CH₂-CH₂-CH₃).

Vorteilhaft ist die überraschend hohe Selektivität der Alkylierung in der 7-Position am Xanthin, während eine Alkylierung an der 1-Position weitgehend unterbleibt. Ferner können die Verbindungen der Formeln III und/oder IV sowie gegebenenfalls der Formel V leicht von der wäßrigen Phase getrennt und für weitere Umsetzungen genutzt werden.

Bevorzugte basische Mittel sind Alkalihydroxide und/oder Alkalicarbonate, wie z. B. Natriumhydroxid, Kaliumhydroxid, Natriumhydrogencarbonat, Kaliumhydrogencarbonat, Natriumcarbonat oder Kaliumcarbonat.

Vorteilhaft sind die quartären Ammonium verbindungen der Formel III, die in Wasser nur schwer oder nicht löslich sind. Bevorzugte quartäre Ammonium verbindungen der Formel III sind Methyltrioctylammoniumchlorid, Methyl-trioctylammoniumhydroxid, Methyl-tricaprylammoniumchlorid, Methyl-tricaprylammoniumhydroxid oder Ethyl-trioctylammoniumchlorid.

Bevorzugte Polyether der Formel V sind Ethylenglykoldibutylether, Diethylenglykoldibutylether, Triethylenglykoldibutylether, Tetraethylenglykoldibutylether, Diethylenglykol-ethyl-tert.-butylether, Propylenglykoldibutylether, Dipropylenglykoldibutylether, Polyethylenglykoldibutylether und Polypropylenglykoldibutylether verschiedener Etherkettenlänge (wobei n in der Formel V die Bedeutung 2 bis 8 hat).

Bevorzugte Alkylierungsmittel sind (C₁-C₆)-Alkylhalogenide wie Alkylchlorid, Alkylbromid, Alkylfluorid oder Alkyljodid, insbesondere Methylchlorid, Ethylchlorid oder Propylchlorid; (C₁-C₆)-Dialkylsulfat wie Dimethyl-, Diethyl-, Dipropyl-, Dibutyl-, Dipentyl- oder Dihexylsulfat;
oder (C₁-C₆)-Dialkylcarbonat wie Dimethyl-, Diethyl-, Dipropyl-, Dibutyl-, Dipentyl- oder Dihexylcarbonat.

Unter dem Begriff Anion versteht man Chlorid, Bromid, Hydrogensulfat oder Hydroxid.

Unter dem Begriff Alkyl werden Kohlenwasserstoffreste wie Methyl, Ethyl, Propyl, Butyl, Pentyl oder Hexyl verstanden.

3-(C₁-C₆)-Alkylxanthine als Ausgangssubstanzen für die erfindungsgemäße Alkylierungsreaktion sind nach literaturbekannten Verfahren herstellbar, beispielsweise durch modifizierte "Trauben-Synthese" (Ullmann's Enzyklopädie der Technischen Chemie, 4. Auflage, Band 19 (1180) Seite 579).

Bei 3-Alkylxanthin-Salz-Herstellung, geht man so vor, daß zunächst das 3-(C₁-C₆)-Alkylxanthin in wäßriger Phase mit Alkalihydroxid und/oder Alkalicarbonat, wie z. B. Natriumhydroxid, Kaliumhydroxid, Natriumhydrogencarbonat, Kaliumhydrogencarbonat, Natriumcarbonat oder Kaliumcarbonat, in das entsprechende Salz überführt wird. Vorzugsweise verwendet man auf 100 Mol 3-Alkylxanthin 100 bis 120 Mol der vorgenannten Alkalihydroxide oder Alkalicarbonate.

Anschließend gibt man zu der 3-Alkylxanthin-Salzlösung oder -Suspension eine quartäre Ammonium- oder Phosphoniumverbindung der Formel III oder IV zu. Es können auch Mischungen von Ammonium- und/oder Phosphoniumverbindungen verwendet werden. Die Verbindungen der Formel III und/oder IV sind in Wasser nicht oder nur schwer löslich. Daher bildet sich ein Zweiphasengemisch, bestehend aus der wäßrigen Phase und der Phase, die durch die Ammonium- und/oder Phosphoniumverbindungen der Formeln III und IV gebildet wird. Unter dem Begriff Zweiphasengemisch wird die Mischung von zwei flüssigen Phasen - Wasserphase und die Phase, die Ammonium- und/oder Phosphoniumverbindungen der Formeln III und IV enthält - verstanden. Das Zweiphasengemisch enthält in der Regel keine weitere Fest-/Flüssigphasengrenze. Es kann jedoch vorkommen, daß bei niedrigen Temperaturen und hohen Konzentrationen der Xanthinsalze Ausflockungen entstehen. Das Zweiphasengemisch wird nach üblichen Methoden gerührt und gemischt, damit eine gute Verteilung der Phasen gewährleistet wird.

Vorzugsweise verwendet man auf 100 Mol 3-Alkylxanthin der Formel II 5 bis 100 Mol der Verbindung der Formeln III, bevorzugt 10 bis 60 Mol, insbesondere 10 bis 50 Mol.

Gegebenenfalls kann zur weiteren Reaktionsbeschleunigung ein linearer Polyether der Formel V zugesetzt werden. Vorzugsweise verwendet man auf 100 Mol 3-Alkylxanthin der Formel II 3 bis 100 Mol der linearen Polyether der Formel V, bevorzugt 5 bis 80 Mol, insbesondere 10 bis 50 Mol.

Zusätzlich kann zur verbesserten Trennung des Zweiphasengemischs des obengenannten Reaktionsgemisches den Verbindungen der Formeln III und V ein organisches Lösemittel zugesetzt werden, wie z. B. Heptan, Cyclohexan, Dimethylcyclohexan, Butylacetat, Amylacetat, Dioxan, Anisol oder Amylalkohol. Vorzugsweise wird jedoch ohne zusätzliche Lösemittel gearbeitet.

Vorteilhaft ist das zugesetzte Lösungsmittel in Wasser nur schwer oder nicht löslich. Die eingesetzten Mengen der Lösemittel können in weiten Bereichen schwanken und können von einem Fachmann leicht ermittelt werden.

Anschließend wird das Alkylierungsmittel zugefügt. Das molase Verhältnis von 3-Alkylxanthin zu Alkylierungsmittel beträgt 1:1.02 bis 1.5, bevorzugt 1:1,08 bis 1.2. Für die Methylierung von beispielsweise 100 Mol 3-Methylxanthin werden 102 bis 150 Mol Methylchlorid, insbesondere 105 bis 140 Mol, bevorzugt 108 bis 120 Mol benötigt, oder 102 bis 150 Mol Dimethylsulfat, insbesondere 105 bis 130 Mol, bevorzugt 110 bis 120 Mol.

Die Reaktionstemperatur kann in weiten Bereichen schwanken. Sie liegt im allgemeinen bei - 10 bis + 140 °C, vorzugsweise von 40 bis 110 °C.

Bei dem Alkylierungsverfahren mit Methylchlorid erreicht man einen Überdruck im Reaktionsgefäß bis zu 5 bar, so daß die Reaktion in für chemische Reaktionen üblichen Apparaten erfolgen kann.

Die Reaktionszeit liegt im allgemeinen zwischen 1 Stunde und 4 Stunden.

Bei der Alkylierungsreaktion mit Dimethylsulfat wird die sich während der Reaktion bildende Methylschwefelsäure durch langsame Zugabe von Alkalihydroxid, Alkalicarbonat, Erdalkalihydroxid oder Erdalkalicarbonat neutralisiert.

Nach Ablauf der Reaktionszeit werden die Phasen des Zweiphasengemischs voneinander getrennt. Dann wird zunächst das ausgeflockte 3,7-Dialkylxanthin der Formel I mit Alkalihydroxid und/oder Erdalkalihydroxid und/oder Alkalicarbonat versetzt, bis sich eine Emulsion bildet. Dazu benötigt man in der Regel auf die oben beschriebene Ansatzgröße etwa 80 bis 120 Mol.

Die Phasen des Zweiphasengemischs trennt man nun nach üblichen Methoden voneinander ab. Die organische Phase, in der im wesentlichen die Verbindung der Formel III sowie gegebenenfalls die Verbindung der Formel V vorliegen, kann direkt oder nach Waschen mit Wasser erneut für eine Alkylierungsreaktion eingesetzt werden. Die wäßrige Phase, die nun das 3,7-Dialkylxanthin-Salz und die Verunreinigungen enthält, wird mit Filterhilfsmitteln auf Cellulose- oder Kieselsäurebasis oder Aktivkohle versetzt und filtriert. Das Filtrat wird erhitzt und rasch mit einer Mineralsäure bis zu dem für die Abtrennung der Nebenprodukte günstigen pH-Wert versetzt, wobei das reine 3,7-Dialkylxanthin ausgefällt wird. Als Mineralsäuren sind beispielhaft genannt: Salzsäure, Schwefelsäure oder Salpetersäure. Es kann auch Kohlensäure eingesetzt werden.

Die Fälltemperatur liegt im allgemeinen bei etwa 50 bis 110 °C, insbesondere 75 bis 105 °C, vorzugsweise bei 85 bis 95 °C. Nach der Fällung beträgt der pH-Wert der Lösung im allgemeinen 7 bis 10.

Der Verbrauch an Mineralsäure liegt im allgemeinen bei 88 bis 110 Mol.

Der Feststoff wird anschließend durch Absaugen isoliert und zur Entfernung von Nebenprodukten mit Wasser gewaschen.

Das erfindungsgemäße Verfahren wird nun durch die folgenden Beispiele näher erläutert.

### Beispiel 1

In einem Reaktor legt man 2000 ml Wasser, 400 g 3-Methylxanthin, 310 g 33%ige Natronlauge, 20 g Natriumbicarbonat und 200 g Methyl-trioctylammoniumchlorid vor. Nachdem der Reaktor dicht verschlossen ist, leitet man bei ca. 50 °C 135 g Methylchlorid ein. Anschließend läßt man bei ca. 110 °C noch nachreagieren, bis der Innendruck nicht mehr weiter fällt. Der Reaktor wird auf ca. 60 °C gekühlt und kurz evakuiert. Danach setzt man 310 g 33%ige Natronlauge zu und trennt anschließend die organische Phase ab. Diese. wäscht man mit 100 ml Wasser nach. Das so wiedergewonnene Methyl-trioctylammoniumchlorid kann für die nächste Methylierung wieder eingesetzt werden. Man versetzt die wäßrige Phase mit Aktivkohle und filtriert die Lösung. Anschließend fällt man bei 85 bis 95 °C durch Zutropfen von insgesamt 252 g 37%iger Salzsäure das Theobromin aus. Nach Abkühlen saugt man das Theobromin ab und wäscht mit Wasser nach. Nach Trocknen bis zur Gewichtskonstanz erhält man 367 g Theobromin mit einem Gehalt von 99,5 % (HPLC); Schmelzpunkt 350,5 bis 351 °C. Das sind 84,5 % des theoretischen Wertes, bezogen auf das Einsatzprodukt 3-Methylxanthin.

### HPLC-Bestimmung

| Probenvorbereitung | |
|---|---|
| 50 mg | Theobromin (Probe) in |
| 90 ml | Wasser suspendieren, |
| 1 ml | 1 M Natronlauge zusetzen und die Probe klar lösen. Danach mit |
| 2 ml | 1 M Essigsäure die Probe ansäuern und auf 100 ml im Meßkolben mit Wasser auffüllen. Vergleichslösungen: 1 mg Coffein, 3-Methylxanthin und Theophyllin - wie oben beschrieben - lösen. |

| Analysenbedingungen | |
|---|---|
| Einspritzmenge | 10 µl |
| Säule | RP 8, 250 -4, 10 µm ®Lichrosorb (Fa. E. Merck, Darmstadt), |
| Flußrate | 1 ml/min |
| Mobile Phase | 80 Vol-% 0,1 %ige KH₂PO₄ - Lösung 20 Vol-% Methanol |
| Detektion | UV-Detektor 273 nm |
| Laufzeit | 20 min |
| Retentionszeit | 3-Methylxanthin 4,05 min, Theobromin 5,47 min Coffein 11,15 min |

### Fließpunktbestimmung

Die Fließpunkte wurden durch Differentialthermoanalyse mit dem System Mettler TA3000 bestimmt.

### Beispiel 2

In einem Reaktor legt man 2000 ml Wasser, 400 g 3-Methylxanthin, 310 g 33%ige Natronlauge, 20 g Natriumbicarbonat und 200 g Methyl-trioctylammoniumchlorid und 100 g Diethylenglykoldibutylether vor. Nachdem der Reaktor dicht verschlossen ist, leitet man bei ca. 50 °C 134 g Methylchlorid ein. Anschließend läßt man bei ca. 90 °C noch nachreagieren, bis der Innendruck nicht mehr weiter fällt. Der Reaktor wird auf ca. 50 °C gekühlt und kurz evakuiert. Danach setzt man 310 g 33%ige Natronlauge zu und trennt anschließend die organische Phase ab. Diese wäscht man mit 100 ml Wasser nach. Das so wiedergewonnene Methyl-trioctylammoniumchlorid und Diethylenglykoldibutylether kann für die nächste Methylierung wieder eingesetzt werden. Die wäßrige Phase versetzt man mit Aktivkohle und filtriert die Lösung. Anschließend fällt man bei 85 bis 95 °C durch Zutropfen von insgesamt etwa 252 g 37%iger Salzsäure das Theobromin aus. Nach Abkühlen saugt man das Theobromin ab und wäscht mit Wasser nach. Nach Trocknen bis zur Gewichtskonstanz erhält man 376 g Theobromin mit einem Gehalt von 99,5 % (HPLC). Das sind 86,5 % des theoretischen Wertes bezogen auf das Einsatzprodukt 3-Methylxanthin.

### Beispiel 3

In einer Rührapparatur legt man 2000 ml Wasser, 400 g 3-Methylxanthin, 294 g 33%ige Natronlauge, 200 g Methyl-trioctylammoniumchlorid und 100 g Triethylenglykoldibutylether vor. Anschließend tropft man bei ca. 50 bis 60 °C 351 g Dimethylsulfat zu. Gegen Ende der Zugabe tropft man zusätzlich 16 g 33%ige Natronlauge zum Reaktionsgemisch hinzu. Anschließend läßt man bei ca. 60 °C noch nachreagieren. Man kühlt ab und setzt danach 310 g 33%ige Natronlauge zu. Anschließend trennt man die organische Phase ab. Diese wäscht man mit 100 ml Wasser nach. Das so wiedergewonnene Methyl-trioctylammoniumchlorid und Triethylenglykoldibutylether kann für die nächste Methylierung wieder eingesetzt werden. Die wäßrige Phase wird mit Aktivkohle versetzt und filtriert. Anschließend fällt man bei 85 bis 95 °C durch Zutropfen von insgesamt etwa 250 g 37%iger Salzsäure das Theobromin aus. Nach Abkühlen saugt man das Theobromin ab und wäscht mit Wasser nach. Nach Trocknen bis zur Gewichtskonstanz erhält man 361 g Theobromin mit einem Gehalt von 99,5 % (HPLC). Das sind 83,2 % des theoretischen Wertes bezogen auf das Einsatzprodukt 3-Methylxanthin.

## Patentansprüche

1. Verfahren zur Gewinnung von 3,7-Dialkylxanthinen der Formel I 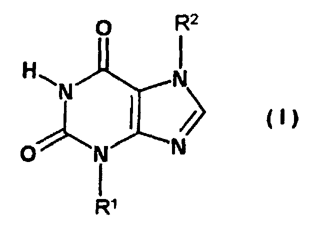 wobei R¹ und R² unabhängig voneinander für (C₁-C₆)-Alkyl, geradkettig oder verzweigt, stehen,
aus 3-Alkylxanthin der Formel II 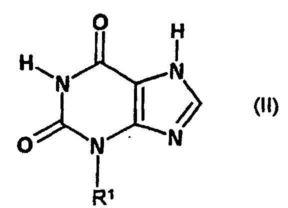 in der R¹ für (C₁-C₆)-Alkyl, geradkettig oder verzweigt steht,
daß zuerst in wäßriger Phase mit einem basischen Mittel in sein Salz überführt und anschließend alkyliert wird,
**dadurch gekennzeichnet, daß** man das erhaltene Salz in Gegenwart von mindestens einer in Wasser schwer oder nicht löslichen quartären Ammoniumverbindung der Formel III, 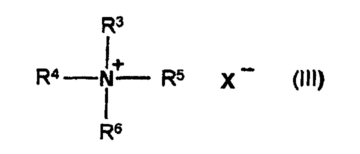 wobei R³ bis R⁶ gleich oder verschieden sind und unabhängig voneinander für
a) (C₁-C₂₀)-Alkyl, geradkettig oder verzweigt,
b) Benzyl oder
c) Phenyl stehen und
X für Anion aus der Gruppe Chlorid, Bromid, Hydrogensulfat oder Hydroxid steht,
in einem Zweiphasengemisch, enthaltend zwei flüssige Phasen, worin die eine Phase Wasser und die andere Phase die quarternäre Ammoniumverbindung der Formel III enthält,
mit einem Alkylierungsmittel, welches 1 bis 6 Kohlenstoffatome im Alkylteil hat, umsetzt und
auf 100 Mol 3-Alkylxanthin der Formel II 5 bis 100 Mol der quartären Ammoniumverbindung der Formel III eingesetzt werden.

2. Verfahren nach Anspruch 1, **dadurch gekennzeichnet, daß** man die Alkylierung in Gegenwart eines linearen Polyethers der Formel V
R¹¹-O-(Y)ₙ-R¹² (V)
durchführt, wobei
R¹¹ und R¹² gleich oder verschieden sind und unabhängig voneinander für (C₁-C₈)-Alkyl stehen,
Y für einen Rest aus der Gruppe
a) -CH₂-CH₂-O- oder
b) -CH₂-CH₂-CH₂-O-
und n für eine ganze Zahl von 1 bis 8 steht.

3. Verfahren nach Anspruch 1 oder 2, **dadurch gekennzeichnet, daß** man ein 3,7-Dialkylxanthin der Formel 1, wobei R¹ und R² unabhängig voneinander für (C₁-C₃)-Alkyl, stehen, herstellt.

4. Verfahren nach Anspruch 1, 2 oder 3, **dadurch gekennzeichnet, daß** man 3,7-Dimethylxanthin, 3-Ethyl-7-propylxanthin oder 3-Methyl-7-propylxanthin herstellt.

5. Verfahren nach einem oder mehreren der Ansprüche 1 bis 4, **dadurch gekennzeichnet, daß** man als basisches Mittel Alkalihydroxid und/oder Alkalicarbonat wie Natriumhydroxid, Kaliumhydroxid, Natriumhydrogencarbonat, Kaliumhydrogencarbonat, Natriumcarbonat oder Kaliumcarbonat und als quartäre Ammoniumbindung der Formel III Methyl-trioctyl-ammoniumchlorid, Methyl-trioctylammoniumhydroxid, Methyl-tricapryl-ammoniumchlorid, Methyl-tricaprylammoniumhydroxid, Ethyl-trioctylammoniumchlorid und als Polyether der Formel V Ethylenglykoldibutylether, Diethylenglykoldibutylether, Triethylenglykoldibutylether, Tetraethylenglykoldibutylether, Diethylenglykol-ethyltert.-butylether, Propylenglykoldibutylether, Dipropylenglykoldibutylether, Polyethylenglykoldibutylether oder Polypropylenglykoldibutylether einsetzt.

6. Verfahren nach einem oder mehreren der Ansprüche 1 bis 5, **dadurch gekennzeichnet, daß** man als Alkylierungsmittel (C₁-C₆)-Alkylhalogenid, (C₁-C₆)-Dialkylsulfat oder (C₁-C₆)-Dialkylcarbonat, insbesondere Methylchlorid, Ethylchlorid, Propylchlorid oder Dimethylsulfat, einsetzt.

7. Verfahren nach einem oder mehreren der Ansprüche 1 bis 6, **dadurch gekennzeichnet, daß** man auf 100 Mol 3-Alkylxanthin der Formel II 10 bis 60 Mol der Verbindung der Formel III, bevorzugt 10 bis 50 Mol, einsetzt und gegebenenfalls 3 bis 100 Mol des linearen Polyethers der Formel V, bevorzugt 5 bis 80 Mol, insbesondere 10 bis 50 Mol, zufügt.

8. Verfahren nach einem oder mehreren der Ansprüche 1 bis 7, **dadurch gekennzeichnet, daß** man die Alkylierung bei Temperaturen von- 10 bis + 140 °C, bevorzugt von 40 bis 110 °C, durchführt.

9. Verfahren nach einem oder mehreren der Ansprüche 1 bis 8, **dadurch gekennzeichnet, daß** das molare Verhältnis von 3-Alkylxanthin zu Alkylierungsmittel 1 : 1,02 bis 1,5 beträgt, bevorzugt 1 : 1,08 bis 1,2.

10. Verfahren nach einem oder mehreren der Ansprüche 1 bis 9, **dadurch gekennzeichnet, daß** die Verbindung der Formel III sowie gegebenenfalls der Formel V aus einer vorherigen Umsetzung gemäß Anspruch 1 oder 2 stammt.

## Claims

1. A process for obtaining 3,7-dialkylxanthines of the formula I 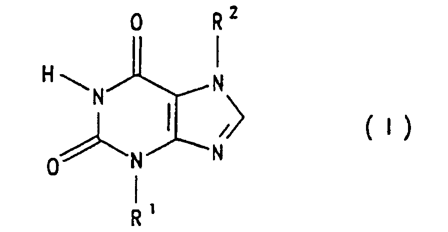 where R¹ and R², independently of each other, are (C₁-C₆)-alkyl, which may be straight-chain or branched, from 3-alkylxanthine of the formula II 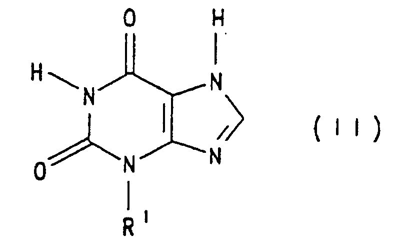 in which R¹ is (C₁-C₆)-alkyl, which may be straight-chain or branched,
which is firstly converted in aqueous phase and
using a basic agent into its salt and is then alkylated,
wherein the resulting salt is reacted in a two-phase mixture, containing two liquid phases in which one phase contains water and the other phase contains the quaternary ammonium compound of the formula III, with an alkylating agent which has 1 to 6 carbon atoms in the alkyl moiety in the presence of at least one quaternary ammonium compound of the formula III which is insoluble or sparingly soluble in water 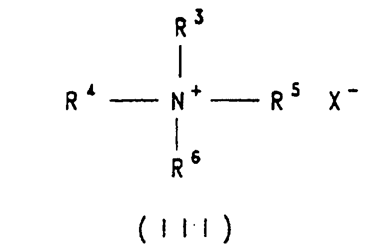 where R³ to R⁶ are identical or different and, independently of each other, are
a) (C₁-C₂₀)alkyl, which may be straight-chain or branched,
b) benzyl or
c) phenyl and
X is an anion from the group comprising chloride, bromide, hydrogen sulfate and hydroxide, and
5 to 100 mol of the quaternary ammonium compound of the formula III are employed per 100 mol of 3-alkylxanthine of the formula 11.

2. The process as claimed in claim 1, wherein the alkylation is carried out in the presence of a linear polyether of the formula V
R¹¹ - O - (Y)ₙ - R¹² (V)
R¹¹ and R¹² xare identical or different and, independently of each other, are (C₁-C₈)-alkyl,
Y is a radical from the group
a) -CH₂-CH₂-O- or
b) -CH₂-CH₂-CH₂-O-
and n is an integer from 1 to 8.

3. The process as claimed in claim 1 or 2, wherein a 3,7-dialkylxanthine of the formula I where R¹ and R², independently of each other, are (C₁-C₃)-alkyl, is prepared.

4. The process as claimed in claim 1, 2 or 3, wherein 3,7-dimethylxanthine, 3-ethyl-7-propylxanthine or 3-methyl-7-propylxanthine is prepared.

5. The process as claimed in one or more of claims 1 to 4, wherein an alkali metal hydroxide and/or alkali metal carbonate, such as sodium hydroxide, potassium hydroxide, sodium hydrogen carbonate, potassium hydrogen carbonate, sodium carbonate or potassium carbonate, is employed as the basic agent, and methyltrioctylammonium chloride, methyltrioctylammonium hydroxide, methyltricaprylammonium chloride, methyltricaprylammonium hydroxide or ethyltrioctylammonium chloride is employed as the quaternary ammonium compound of the formula III, and ethylene glycol dibutyl ether, diethylene glycol dibutyl ether, triethylene glycol dibutyl ether, tetraethylene glycol dibutyl ether, diethylene glycol ethyl tert-butyl ether, propylene glycol dibutyl ether, dipropylene glycol dibutyl ether, polyethylene glycol dibutyl ether or polypropylene glycol dibutyl ether is employed as the polyether of the formula V.

6. The process as claimed in one or more of claims 1 to 5, wherein a (C₁-C₆)-alkyl halide, (C₁-C₆)-dialkyl sulfate or (C₁-C₆)-dialkyl carbonate, in particular methyl chloride, ethyl chloride, propyl chloride or dimethyl sulfate, is employed as the alkylating agent.

7. The process as claimed in one or more of claims 1 to 6, wherein 10 to 60 mol of the compound of the formula III, preferably 10 to 50 mol, are employed per 100 mol of 3-alkylxanthine of the formula II and, where appropriate, 3 to 100 mol of the linear polyether of the formula V, preferably 5 to 80 mol, in particular 10 to 50 mol, are added.

8. The process as claimed in one or more of claims 1 to 7, wherein the alkylation is carried out at temperatures of -10 to +140°C, preferably from 40 to 110°C.

9. The process as claimed in one or more of claims 1 to 8, wherein the molar ratio of 3-alkylxanthine to alkylating agent is 1:1.02 to 1.5, preferably 1:1.08 to 1.2.

10. The process as claimed in one or more of claims 1 to 9, wherein the compound of the formula III, as well as, where appropriate, of the formula V, originate from a previous reaction as claimed in claim 1 or 2.

## Revendications

1. Procédé pour l'obtention de 3,7-dialkylxanthines de formule I 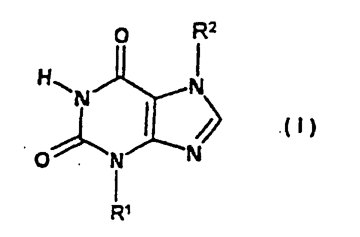 dans laquelle R¹ et R² représentent indépendamment l'un de l'autre un groupe alkyle en C₁ à C₆, à chaîne linéaire ou ramifiée,
à partir de 3-alkylxanthine de formule (II) 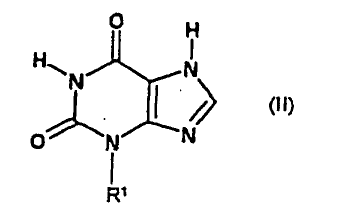 dans laquelle R¹ représente un groupe alkyle en C₁ à C₆, à chaîne linéaire ou ramifiée,
**caractérisé en ce qu'**elle est d'abord transformée en son sel en phase aqueuse avec un agent basique et ensuite alkylée,
**caractérisé en ce qu'**on met à réagir le sel obtenu en présence d'au moins un composé ammonium quaternaire difficilement ou non soluble dans l'eau de formule III, 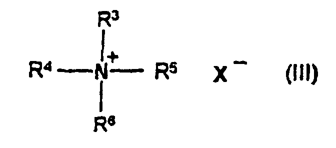 dans laquelle R³ à R⁶ sont identiques ou différents et représentent indépendamment les uns des autres
a) un groupe alkyle en C₁ à C₂₀, linéaire ou ramifié,
b) benzyle ou
c) phényle et
X représente un anion choisi parmi le chlorure, bromure, hydrogénosulfate ou hydroxyde,
dans un mélange à deux phases, contenant deux phases liquides, dans lequel une phase contient de l'eau et l'autre phase le composé ammonium quaternaire de formule III,
avec un agent d'alkylation, qui a dans la partie alkyle de 1 à 6 atomes de carbone et
on utilise pour 100 mol de 3-alkylxanthine de formule II 5 à 100 mol du composé ammonium quaternaire de formule III.

2. Procédé selon la revendication 1, **caractérisé en ce qu'**on réalise l'alkylation en présence d'un polyéther linéaire de formule V
R¹¹-O-(Y)ₙ-R¹² (V),
dans laquelle
R¹¹ et R¹² sont identiques ou différents et représentent indépendamment l'un de l'autre un groupe alkyle en C₁ à C₈,
Y représente un groupe choisi parmi
a) -CH₂-CH₂-O- ou
b) -CH₂-CH₂-CH₂-O-
et n représente un nombre entier de 1 à 8.

3. Procédé selon la revendication 1 ou 2, **caractérisé en ce qu'**on prépare une 3,7-dialkylxanthine de formule I, dans laquelle R¹ et R² représentent indépendamment l'un de l'autre un groupe alkyle en C₁-C₃.

4. Procédé selon la revendication 1, 2 ou 3, **caractérisé en ce qu'**on prépare une 3,7-diméthylxanthine, 3-éthyl-7-propylxanthine ou 3-méthyl-7-propylxanthine.

5. Procédé selon une ou plusieurs des revendications 1 à 4, **caractérisé en ce qu'**on utilise comme agent basique un hydroxyde de métal alcalin et/ou un carbonate de métal alcalin comme l'hydroxyde de sodium, l'hydroxyde de potassium, l'hydrogénocarbonate de sodium, l'hydrogénocarbonate de potassium, le carbonate de sodium ou le carbonate de potassium et comme composé ammonium quaternaire de formule III le chlorure de méthyltrioctylammonium, l'hydroxyde de méthyltrioctylammonium, le chlorure de méthyltricaprylammonium, l'hydroxyde de méthyltricaprylammonium, le chlorure d'éthyl-trioctylammonium et comme polyéther de formule V l'éther dibutylique d'éthylèneglycol, l'éther dibutylique de diéthylèneglycol, l'éther dibutylique de triéthylèneglycol, l'éther dibutylique de tétraéthylèneglycol, l'éther éthyl-tert.-butylique de diéthylèneglycol, l'éther dibutylique de propylèneglycol, l'éther dibutylique de dipropylèneglycol, l'éther dibutylique de polyéthylèneglycol ou l'éther dibutylique de polypropylèneglycol.

6. Procédé selon une ou plusieurs des revendications 1 à 5, **caractérisé en ce qu'**on utilise comme agent d'alkylation un halogénure d'alkyle en C₁-C_{6,} un sulfate de dialkyle en C₁-C₆ ou un carbonate de dialkyle en C₁-C₆, en particulier le chlorure de méthyle, le chlorure d'éthyle, le chlorure de propyle ou de sulfate de diméthyle.

7. Procédé selon une ou plusieurs des revendications 1 à 6, **caractérisé en ce qu'**on utilise pour 100 mol de 3-alkylxanthine de formule II 10 à 60 mol du composé de formule III, de préférence 10 à 50 mol et **en ce qu'**on ajoute éventuellement 3 à 100 mol du polyéther linéaire de formule V, de préférence 5 à 80 mol, en particulier 10 à 50 mol.

8. Procédé selon une ou plusieurs des revendications 1 à 7, **caractérisé en ce qu'**on réalise l'alkylation à des températures de -10 à +140°C, de préférence de 40 à 110°C.

9. Procédé selon une ou plusieurs des revendications 1 à 8, **caractérisé en ce que** le rapport molaire de 3-alkylxanthine sur l'agent d'alkylation est de 1 : 1,02 à 1,5, de préférence de 1 : 1,08 à 1,2.

10. Procédé selon une ou plusieurs des revendications 1 à 9, **caractérisé en ce que** le composé de formule III ainsi qu'éventuellement de formule V est issu d'une réaction précédente selon la revendication 1 ou 2.
